# EUROPEAN PATENT SPECIFICATION

(11) **EP 1 346 984 B1**
(45) Date of publication and mention of the grant of the patent: **06.08.2008**
(21) Application number: 02252013.4
(22) Date of filing: 20.03.2002
(51) Int. Cl.: C07D 213/61

(54) **Process for the preparation of 2-chloro-5-methylpyridine-3- carbaldehyde**
Verfahren zur Herstellung von 2-Chlor-5-methylpyridin-3-carbaldehyd
Procédé de préparation du 2-chloro-5-méthylpyridine-3-carbaldéhyde

(43) Date of publication of application: 24.09.2003
(73) Proprietor: COUNCIL OF SCIENTIFIC AND INDUSTRIAL RESEARCH, New Delhi 110 001 (IN)
(72) Inventor: Banda, Gangadasu, Andhra Pradesh (IN); Bhimapaka, Chinaraju, Andhra Pradesh (IN); Rao, Vaidya Jayathirtha, Andhra Pradesh (IN)
(74) Representative: Beresford, Keith Denis Lewis

(56) References cited:
- EP-A- 0 702 003
- METH-COHN, OTTO ET AL: "A versatile new synthesis of quinolines and related fused pyridines. Part 12. A general synthesis of 2-chloropyridines and 2-pyridones" J. CHEM. SOC., PERKIN TRANS. 1 (1984), (6), 1173-82 , XP000602179

## Description

### Field of the invention

The present invention relates to a process for the preparation of 2-chloro-5-methylpyridine-3-carbaldehyde, a useful intermediate for the variety of pharmaceutical and pesticide products. The present invention particularly relates to a process for the preparation of 2-chloro-5-methylpyridine-3-carbaldehyde of formula 1 given below using diphosgene and triphosgene.

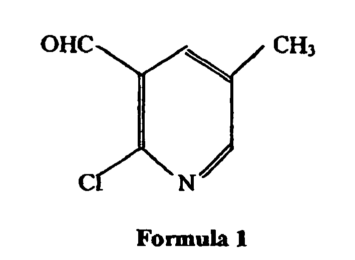

The present invention also relates to a process for the preparation of 2-chloro-5methyl-pyridine-3-carbaldehyde under suitable conditions of mole ratio, time and temperature with high selectivity.

### Background of the invention

Several pyridine, quinoline and isoquinoline compounds are important intermediates in that they have been reported as potential anti tumor agents (J. Med. Chem, 19, 1209, 1976; CA 85: 116544q). Carbaldehyde based products served as ulcer healing agents (Ger. Offen DE 3324034, 1984; CA 101:54936g), allergy inhibitors (EP 120483, 1984; CA 102:95649e; EP120484, 1984; CA 102:113500f), antiviral agents (CA 113:172015b), and useful as intermediates for pharmaceuticals (Ger. Offen DE 4429465, 1996; CA 124:343116u).

Substituted pyridine carbaldehyde is an important compound to prepare the amlexanox a drug which is useful for treatment of mucosites and in the treatment of aphthous ulcers (Drugs of the future 25(5), 511,200).

Chloropyridinecarbaldehyde is a very useful intermediate to make various compounds like sulfones and vinyl pyridines in organic syntheses (Heterocyclic Commun, 5(3), 257,1999) 1,2,3,4-tetrahydrodibenzo-1, 8-naphthyridines are prepared from 2-chloro-3-formyl quinolines (Hetero-cyclic Commun. 6(1),63,2000).

There is a method in the literature for the preparation of 2-chloro-5-methylpyridine-3-carbaldehyde in 12% yield (J.C.S. Perkin Trans. 1,1173, 1984).

European Patent Application No. 0702003 discloses processes for the preparation of 2-halogenopyridine aldehydes from the reaction of cyanoolefins, aminocarbonylolefins or hydroxyiminoolefins with a Vilsmeyer reagent.

### Objects of the invention

The main object of the invention is to provide an improved method for the preparation of 2-chloro-5-methylpyridine-3-carbaldehyde in improved yield.

It is another object of the invention to provide an improved method for the preparation of 2-chloro-5-methylpyridine-3-carbaldehyde with shorter reaction times and suitable temperatures.

It is another object of the invention to provide an improved method for the preparation of 2-chloro-5-methylpyridine-3-carbaldehyde wherein product isolation is very easy.

It is another object of the invention to provide an improved method for the preparation of 2-chloro-5-methylpyridine-3-carbaldehyde using easily handled non-toxic diphosgene and triphosgene.

### Summary of the invention

The present invention describes an improved process for the preparation of 2-chloro-5-methylpyridine-3-carbaldehyde from N-benzyl-N-(1-Propenyl) acetamide using diphosgene and dimethylformamide or triphosgene and dimethylformamide (Vilsmeyer reagent) in 92% yield against reported 12% yield. The present invention has the advantage of using diphosgene and triphosgene instead of toxic phosgene gas and phosphorousoxytrichloride, thereby avoiding the formation of phosphorous salt.

Accordingly the present invention provides a process for the preparation of 2-chloro-5-methylpyridine-3-carbaldehyde of the formula 1 below 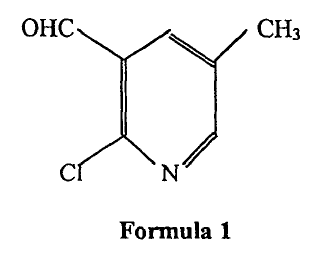 comprising reacting N-benzyl-N-(1-Propenyl) acetamide with a reagent selected from dimethylformamide mixed with diphosgene or triphosgene (Vilsmeyer reagent) under dry conditions at a temperature in the range of 75-100°C.

In another embodiment of the invention, the reaction is carried out for a time period in the range of 4-16 hours.

In another embodiment of the invention, the mole ratio of dimethylformamide to diphosgene or triphosgene is in the range of 1:1 - 2.

In a further embodiment of the invention, the mole ratio of dimethylformamide to diphosgene is in the range of 1:1 - 2.

In a further embodiment of the invention, the mole ratio of dimethylformamide to triphosgene is 1:1.

In a further embodiment of the invention, N-benzyl-N-(1-Propenyl) acetamide and reagent are mixed before heating in an ice cold bath.

In another embodiment of the invention, the mole ratio of diphosgene and triphosgene with dimethylformamide is critical to the formation of the formylating reagent and subsequent formation of the cyclised product leading to 2-chloro-5-methylpyridine-3-carbaldehyde.

Preferably, the weight proportions of the cyclising reagent to the acetamide are in the range 70 to 80: 5 to 6.

### Detailed description of the invention

The following examples are given by way of illustration of the present invention and there fore should not be construed to the scope of the present invention.

### Example 1:

Dimethylformamide (13.54 g, 0.185 moles) was added to a well stirred and cooled material of diphosgene (36.64 g, 0.185 moles) at 4°C in 30 minutes in an ice bath followed by N-benzyl-N-(1-Propenyl) acetamide (5g, 0.026 moles) at the same temperature. The reaction mixture was further continued for 2 hours at 25°C. The ice cold bath was removed and heated to 75°C for 5 hours. The orange-yellow coloured organic mass was poured in ice cold water (200g) with stirring. The mass was extracted with methylenechloride (2x200ml) and the layer was separated. The organic layer was dried over sodium sulfate, and the solvent was removed under reduced pressure. The obtained residue was subjected to chromatographic purification on silica gel to give 2-chloro-5-methylpyridine-3-carbaldehyde (3.78 g) in 92% yield.

### Example 2:

Dimethylformamide (13.54 g, 0.185 moles) was added to a well stirred and cooled material of diphosgene (36.64g, 0.185moles) at 10°C in 30 minutes in an ice bath followed by N-benzyl-N-(1-Propenyl) acetamide (5g, 0.026 moles) at the same temperature. The reaction mixture was further continued for 2 hours at 25°C. The ice cold bath was removed and heated to 75°C for 5 hours. The orange-yellow coloured organic mass was poured in ice cold water (200g) with stirring. The mass was extracted with methylenechloride (2x200ml) and the layer separated. The organic layer was dried over sodium sulfate, and solvent removed under reduced pressure. Residue obtained was subjected to chromatographic purification on silica gel to give 2-chloro-5-methylpyridine-3-carbaldehyde (3.78 g) in 92% yield.

### Example 3:

Dimethylformamide (19.34g, 0.264 moles) was added to a well stirred and cooled material of diphosgene (36.64 g, 0.185 moles) at 4°C in 30 minutes in an ice bath followed by N-benzyl-N-(1-Propenyl) acetamide (5g, 0.026 moles) at the same temperature. The reaction mixture was further continued for 2 hours at 30°C. The ice cold both was removed and heated to 75°C for 8 hours. Work up procedure was carried out according to the procedure of example 1. Before that excess DMF was removed under reduced pressure. The obtained residue was subjected to chromatographic purification on silica gel to give 2-chloro-5-methylpyridine-3-carbaldehyde (3.78g) in 92% yield.

### Example 4:

Dimethylformamide (19.34g, 0.264 moles) was added to a well stirred and cooled material of diphosgene (36.64 g, 0.185 moles) at 10°C in 30 minutes in an ice bath followed by N-benzyl-N-(1-Propenyl) acetamide (5g, 0.026 moles) at the same temperature. The reaction mixture was further continued for 2 hours at 30°C. The ice cold both was removed and heated to 75°C for 8 hours. Work up procedure was carried out according to the procedure of example 1. Before that excess DMF was removed under reduced pressure. The obtained residue was subjected to chromatographic purification on silica gel to give 2-chloro-5-methylpyridine-3-carbaldehyde (3.78g) in 92% yield.

### Example 5:

Dimethylformamide (2.71g, 0.037 moles) was added to a well stirred and cooled material of triphosgene (10.99g, 0.037moles) at 4°C in 30 minutes in an ice bath followed by N-benzyl-N-(1-Propenyl) acetamide (1g, 0.005 moles) at the temperature. The reaction mixture was further continued for 2 hours at 25°C. The ice cold bath was removed and heated to75°C for 5 hours. Work up procedure was carried out according to the procedure of example 1. The obtained residue was subjected to chromatographic purification on silica gel to give 2-chloro-5-methylpyridine-3-carbaldeyde in 92% yield.

### Example 6:

Dimethylformamide (13.54 g, 0.185 moles) was added to well stirred and cooled material of triphosgene (55g, 0.185 moles) at 4°C in 30 minutes in an ice bath followed by N-benzyl-N-(1-Propenyl) acetamide (5g, 0.02645 moles) at the same temperature. The reaction mixture was further continued for 2 hours at 25°C. The ice cold bath was removed and heated to 75°C for 5 hours. The work up procedure was carried out according to the above mentioned procedure. The obtained residue was subjected to chromatographic purification on silica gel to give 2-choloro-5-methylpyridine-3-carbaldehyde in 92% yield.

### Advantages:

- High yield of 2-choloro-5-mothylpyridine-3-carbaldehyde has been obtained for the first time.
- The advantage of the present invention is the employment of controlled time and temperature conditions during the reaction which are critical to the formation of the reagent and the product.
- Another advantage of the present invention is that it requires shorter reaction times and suitable temperatures.
- Another advantage of the present invention is that the product isolation is very easy.
- Yet another advantage of the present invention is that the handling of diphosgene and triphosgene are very easy when compared to toxic phosgene gas.

## Claims

1. A process for the preparation of 2-chloro-5-methylpyridine-3-carbaldehyde of the formula 1 below 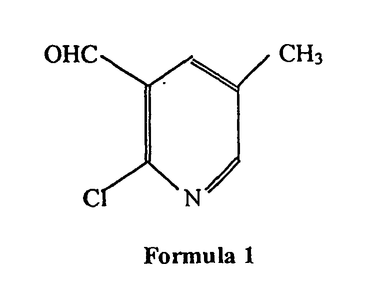 comprising reacting N-benzyl-N-(1-Propenyl) acetamide with a reagent selected from dimethylformamide mixed with diphosgene or triphosgene (Vilsmeyer reagent) under dry conditions at a temperature in the range of 75-100°C.

2. A process as claimed in claim 1 wherein the reaction is carried out for a time period in the range of 4-16 hours.

3. A process as claimed in claim 1 or claim 2 wherein the mole ratio of dimethylformamide to diphosgene or triphosgene is in the range of 1:1 - 2.

4. A process as claimed in claim 3 wherein the mole ratio of dimethylformamide to diphosgene is in the range of 1:1 - 2.

5. A process as claimed in claim 3 wherein the mole ratio of dimethylformamide to triphosgene is 1:1.

6. A process as claimed in any preceding claim wherein the N-benzyl-N-(1-Propenyl) acetamide and reagent are mixed before heating in an ice cold bath.

## Patentansprüche

1. Verfahren zur Herstellung von 2-Fluor-5-methylpyridin-3-carbaldehyd der Formel 1 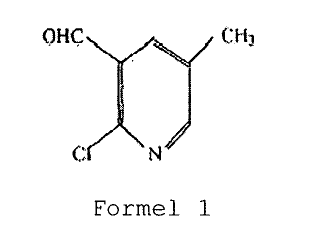 welches das Umsetzen von N-Benzyl-N-(1-propenyl)acetamid mit einem Reagens, ausgewählt unter Dimethylformamid im Gemisch mit Diphosgen oder Triphosgen (Vilsmeyer-Reagens), unter trockenen Bedingungen bei einer Temperatur im Bereich von 75 bis 100°C umfasst.

2. Verfahren nach Anspruch 1, wobei die Reaktion über einen Zeitraum im Bereich von 4 bis 16 Stunden durchgeführt wird.

3. Verfahren nach Anspruch 1 oder 2, wobei das Molverhältnis von Dimethylformamid zu Diphosgen oder Triphosgen im Bereich von 1:1 bis 2 ist.

4. Verfahren nach Anspruch 3, wobei das Molverhältnis von Dimethylformamid zu Diphosgen im Bereich von 1:1 bis 2 ist.

5. Verfahren nach Anspruch 3, wobei das Molverhältnis von Dimethylformamid zu Triphosgen 1:1 ist.

6. Verfahren nach einem der vorstehenden Ansprüche, wobei das N-Benzyl-N-(1-propenyl)acetamid und das Reagens vor dem Erwärmen in einem eisgekühlten Bad gemischt werden.

## Revendications

1. Procédé pour la préparation de 2-chloro-5-méthyl-pyridine-3-carbaldéhyde répondant à la formule 1 ci-dessous 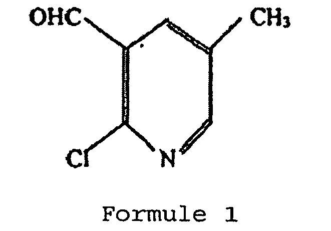 comprenant la réaction de N-benzyl-N-(1-propényl)acétamide avec un réactif choisi entre le diméthylformamide en mélange avec du diphosgène et le diméthylformamide en mélange avec du triphosgène (réactif de Vilsmeyer) dans des conditions anhydres et à une température comprise dans l'intervalle de 75 à 100°C.

2. Procédé suivant la revendication 1, dans lequel la réaction est conduite pendant une période de temps comprise dans l'intervalle de 4 à 16 heures.

3. Procédé suivant la revendication 1 ou la revendication 2, dans lequel le rapport molaire du diméthylformamide au diphosgène ou triphosgène est compris dans l'intervalle de 1:1-2.

4. Procédé suivant la revendication 3, dans lequel le rapport molaire du diméthylformamide au diphosgène est compris dans l'intervalle de 1:1-2.

5. Procédé suivant la revendication 3, dans lequel le rapport molaire du diméthylformamide au triphosgène est égal à 1:1

6. Procédé suivant l'une quelconque des revendications précédentes, dans lequel le N-benzyl-N-1-(1-propényl)acétamide et le réactif sont mélangés avant chauffage dans un bain refroidi par de glace.
